# EUROPÄISCHE PATENTSCHRIFT

(11) **EP 0 982 069 B1**
(45) Veröffentlichungstag und Bekanntmachung des Hinweises auf die Patenterteilung: **29.10.2003**
(21) Anmeldenummer: 99114905.5
(22) Anmeldetag: 30.07.1999
(51) Int. Cl.: B01J 8/00, B01J 8/24, B01D 46/24, B01D 46/44, C07C 17/156, C07C 19/045

(54) **Verfahren zur Feinstaubausschleusung aus einem Wirbelschichtreaktor zur Oxichlorierung von Ethylen**
Method of evacuating fine dust particles from a fluidized bed reactor for oxychlorination of ethylene
Procédé pour evacuer de poussières fines d'un réacteur a lit fluidisée pour l' oxychloration de l'éthylène

(30) Priorität: 21.08.1998 DE 19837957
(43) Veröffentlichungstag der Anmeldung: 01.03.2000
(73) Patentinhaber: Uhde GmbH, 44141 Dortmund (DE)
(72) Erfinder: Benje, Michael, Dr., 64289 Darmstadt (DE)
(74) Vertreter: Patentanwälte Meinke, Dabringhaus und Partner

(56) Entgegenhaltungen:
- DE-A- 19 753 165
- GB-A- 663 752
- US-A- 4 161 389
- US-A- 4 670 993
- US-A- 4 946 654

## Beschreibung

Die Erfindung richtet sich auf ein Verfahren zur Feinstaubausschleusung aus einem Wirbelschichtreaktor zur Oxichlorierung von Ethylen.

Es sind eine Reihe von Prozessen bekannt, bei denen chemische Reaktionen über Katalysatoren in einer Wirbelschicht ausgelöst werden, so beispielsweise bei der Oxichlorierung, bei der Ethylen-Sauerstoff und HCl in einem Wirbelschichtreaktor an einem kupferhaltigen Katalysator zu 1,2-Dichlorethan und Wasser umgesetzt werden. Bei derartigen Wirbelschichtprozessen wird zwangsläufig ein Abrieb der Wirbelschichtpartikel erzeugt, der als Feinstaub in der Wirbelschicht vorliegt. Da diese Feinstaubpartikel durch das Reaktionsgasgemisch mitgerissen werden, können sie innerhalb oder außerhalb des Reaktors beispielsweise durch in Reihe geschaltete Zyklone abgeschieden werden oder auch durch Feinstaubfilter, z.B. durch Schläuche aus einer Gare-Tex-Membran auf PTFE-Nadelfilz.

Aus der älteren, nicht vorveröffentlichten DE-197 53 165-A, der US-4 670 933, der DE-OS 20 19 210 wie auch aus der DE-40 30 086-C1 sind Wirbelschichtreaktoren bzw. -verfahren bekannt, mit innerhalb des Reaktors angeordneten Filterkerzen für das Abgas. Solche Filterkerzen lassen grundsätzlich geringe Mengen an Feinstaub durch (vgl. gutachtlich DE-21 66 912-A1, insbesondere den letzten Absatz der Beschreibung dieser Druckschrift).

Zum technischen Umfeld der Erfindung können auch die GB-633 752, die US-4 161 389 oder die US-4 946 654 gezählt werden.

Aufgrund von physikalischen Gegebenheiten der eingesetzten Wirbelschichten müssen Zykloneinlässe in einer bestimmten Mindesthöhe oberhalb der Wirbelschicht angeordnet sein, da ab dieser Höhe der Staubgehalt im abgezogenen Gas etwa konstant ist, wobei als Beispiel etwa 25 % der Bauhöhe des Reaktors angegeben sei, ohne daß die Erfindung auf diese Maße beschränkt wäre. Es sind Bestrebungen bekannt, die Zyklone und die nachgeschalteten Feinstaubfilter durch einen einzigen unmittelbar im Reaktor angeordneten Feinstaubfilter zu ersetzen, wobei über Druckgaspulse von der Reihenseite her die eingesetzten Filterkerzen abgereinigt werden können. Dabei können die Filter, die eingesetzt werden, unmittelbar in die Wirbelschicht eintauchen, was zwangsläufig zu einer Verringerung der Bauhöhe der Reaktoren führen kann. Auch soll eine solche Verfahrensweise eine vollständige Staubabscheidung ermöglichen.

Ein besonderer Nachteil dieser Vorgehensweise besteht aber darin, daß sich die Wirbelschicht zwangsläufig mit Feinstaubanteilen anreichert, die von der gewünschten sphärischen Form der Wirbelschichtpartikel zwangsläufig erheblich abweichen, so daß sich die Charakteristika der Wirbelschicht ändern. Ist ein geringer Anteil an Kleinstmengen zur besseren Fluidisierung des Wirbelbettes von Nutzen, können größere Mengen einen Übergang von einer sprudelnden Wirbelschicht zu einer stoßenden Wirbelschicht oder zur Belegung der Kühlflächen führen, was zu einer allmählichen Verschlechterung und schließlich zum Zusammenbruch der Wärmeübertragung führt.

Hier setzt die vorliegende Erfindung ein, deren Aufgabe es ist, unter Einsparung von Bauhöhe eines Reaktors eine gezielte Steuerung der Feinstaubpartikelmenge im Reaktor zu ermöglichen bei gleichzeitiger Abfilterung des Feinstaubes aus dem zur Quenche führenden Hauptstrom.

Mit einem Verfahren der eingangs bezeichneten Art wird diese Aufgabe gemäß der Erfindung dadurch gelöst, daß der Feinstaub innerhalb des Reaktors über Filterkerzen, insbesondere Sintermetallfilterkerzen, einer definierten größeren Porenweite als diejenige des Feinstaubfilters ausgeschleust und das Reaktionsgasgemisch zur Quenche aus dem Reaktordom geleitet wird, wobei ein Teilstrom als Bypass-Strom mit einem vorbestimmten Feinstaubanteil unterhalb einer vorbestimmten Korngröße neben dem Hauptstrom aus dem Reaktor ausgeschleust wird.

Mit der erfindungsgemäßen Verfahrensweise ist es möglich, genau den gewünschten Feinstaubanteil im Wirbelschichtreaktor einzustellen. Wird beispielsweise durch eine Siebanalyse einer Katalysatorprobe festgestellt, daß der Feinstaubanteil im Reaktor über ein vorbestimmbares, zulässiges Maß ansteigt, können die Bypass-Leitung und die zugeordneten Filterkerzen geöffnet werden, Feinstaubanteile unterhalb einer bestimmten Mindestkorngröße verlassen damit allmählich den Reaktor.

In weiterer Ausgestaltung ist vorgesehen, daß der Hauptstrom und der Bypass-Strom aus getrennten Domräumen des Reaktors ausgeschleust werden, wobei statt getrennter Domräume auch Gruppen von Filtergrundplatten mit Filterkerzen vorgesehen sein können, die dann unterschiedlich angesteuert werden.

Der Bypass-Strom kann aufgrund unterschiedlicher Kriterien zu- bzw. abgeschaltet werden, beispielsweise aufgrund einer entsprechenden schon erwähnten Analyse einer Katalysatorprobe, aufgrund der Änderung der Wärmeübertragung oder der Verschlechterung des Fluidisierungsverhaltens der Wirbelschicht, z.B. wenn eine annormale hohe Dichte der Wirbelschicht festgestellt wird.

Zur Lösung der gestellten Aufgabe sieht die Erfindung auch einen Wirbelschichtreaktor, insbesondere zur Oxichlorierung von Ethylen, vor unter Einsatz eines dem Abrieb unterworfenen Katalysatorgranulates, z.B. γ-Aluminium-Oxid mit einer mittleren Korngröße von ca. 40 bis 60 µm, der sich dadurch auszeichnet, daß im Dom des Reaktors wenigstens eine Grundplatte mit Filterkerzen, insbesondere Sintermetallfilterkerzen, vorgesehen ist, wobei ggf. die Filterkerzen in den oberen Bereich der Wirbelschicht eintauchen und daß der Domraum oberhalb der die Filterkerzen auf seiner Unterseite tragenden Platte in wenigstens zwei Kammern mit je einem Ausgang für einen Hauptstrom zur Quenche und einem Bypass-Strom geteilt ist.

An dieser Stelle sei bemerkt, daß die Filterkerzen aus einem anderen Material, z.B. Keramik, bestehen können, die Erfindung ist hier nicht auf die genannten Sintermetallfilterkerzen beschränkt. Die Einteilung der die Filterkerzen tragenden Platte in zwei Bereiche, die im Domraum voneinander getrennt sind, hat den Vorteil, daß der Bypass-Strom ohne große Beeinflussung der sonstigen Filter getrennt zu- und abgeschaltet werden kann.

Statt einer solchen Einteilung kann auch vorgesehen sein, die Filterkerzen zu bündeln, zusammenzufassen und sie von außen in den Domraum einzusetzen und dort anzuflanschen. Ein solches Bündel kann dann beispielsweise als Bypass geschaltet sein.

Die Erfindung sieht auch vor, daß die dem Bypass zugeordneten Filterelemente eine gegenüber den Feinstaubfilterkerzen abweichende Porengröße aufweisen zum gezielten Durchlaß von Feinstaubanteilen.

Das Verhältnis an Feinstaub durchlassenden Filterelementen zu Feinstaub rückhaltenden Filterelementen kann im Bereich von 1:9 liegen, worauf die Erfindung auf diese Zahlen aber nicht beschränkt ist, hier können auch andere Verhältnisse vorgesehen sein.

Die die Filterkerzen tragende Platte bzw. Platten können mit einer Abreinigungseinrichtung mittels Druckgaspulsen versehen sein.

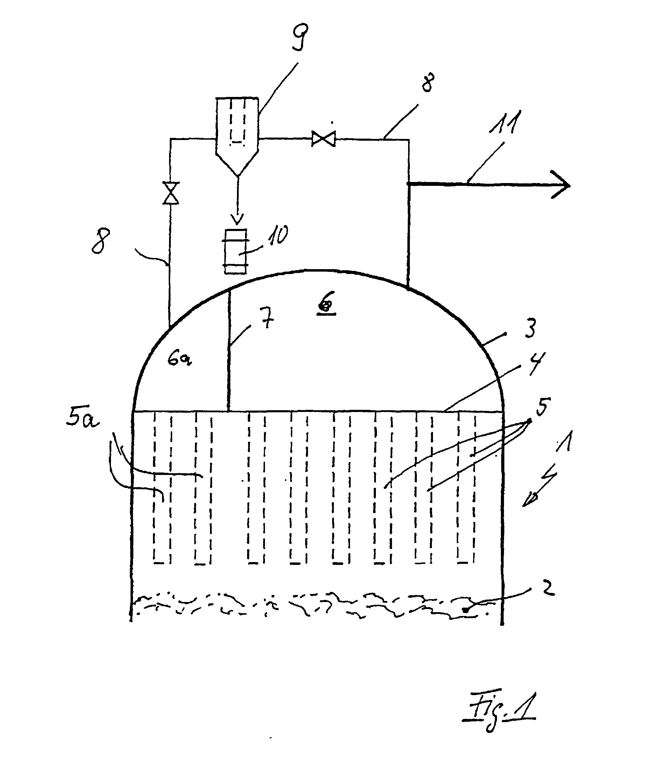

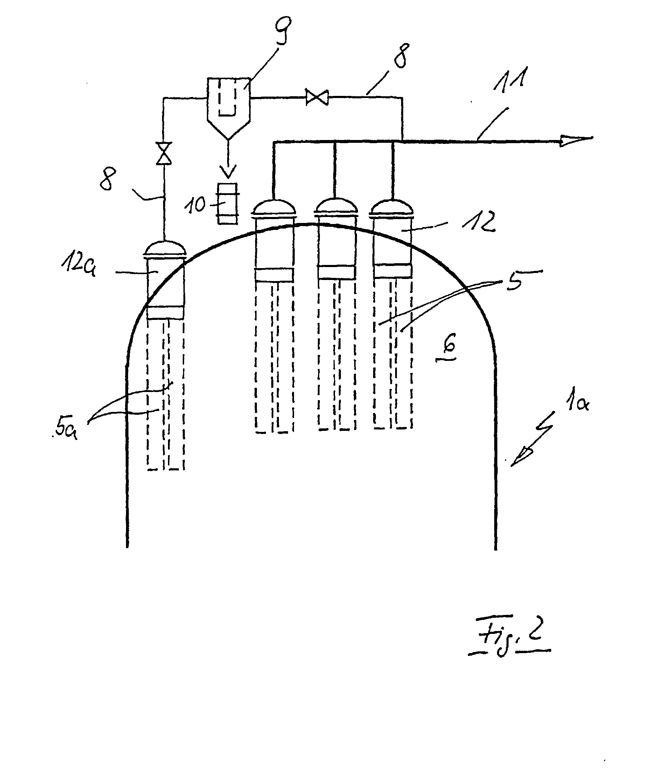

Die Erfindung ist nachstehend anhand der Zeichnung beispielsweise näher erläutert. Diese zeigt in den
- Fig. 1 und 2: stark vereinfachte Ausschnitte aus Reaktordomräumen mit Varianten von erfindungsgemäßer Zuordnung von Filterkerzen.

Ein in den Fig. nur vereinfacht wiedergegebener Reaktor, allgemein mit 1 bezeichent, weist nur mit seiner oberen Grenze eine in Fig. 1 angedeutete Wirbelschicht 2 auf, in der sich beispielsweise γ-Aluminium-Oxid-Partikel mit einer mittleren Korngröße von 40 bis 60 µm befinden, um Ethylen-Sauerstoff und HCl zu 1,2-Dichlorethan und Wasser umzusetzen.

Der Wirbelschichtreaktor 1 weist in seinem mit 3 bezeichneten Dom im Beispiel der Fig. 1 eine Domplatte 4 auf, die eine Vielzahl von Filterkerzen 5 trägt, die auf die Wirbelschicht 2 zuweisen, je nach Bauart ggf. auch in die Wirbelschicht 2 eintauchen können.

Im Beispiel der Fig. 1 ist der mit 6 bezeichnete Domraum durch eine Trennwand 7 in einen größeren Bereich und einen kleineren Bereich 6a getrennt, wobei dem Bereich 6a eine geringe Anzahl von Filterkerzen 5a zugeordnet sind, die, anders als die Filterkerzen 5, Porengrößen aufweisen, die einen Austrag von Feinstaub ermöglichen.

Über eine Bypass-Leitung 8 wird ein entsprechender Gasstrom mit Feinstaubpartikeln, z.B. einem außen liegenden Feinstaubfilter 9, zugeführt, der Feinstaub kann dann ausgetragen und entsorgt werden, was mit einem Behälter 10 angedeutet ist, das Gas kann über die Bypass-Leitung 8 dann wieder dem Hauptstrom, mit 11 bezeichnet, zugeführt werden.

In Fig. 2 ist eine Variante dargestellt, hier sind in dem Domraum 6 von außen Gruppen von Filterelementen 12 eingesetzt, eine Nebengruppe mit 12a bezeichnet ist der Bypass-Leitung zugeordnet. Die im wesentlichen gleichen Teile in Fig. 2 tragen dasselbe Bezugszeichen wie bei der Ausführungsform gemäß Fig. 1, soweit nicht Besonderheiten gegeben sind.

Auch hier können wieder die Filterkerzen 5a eine so große Porenweite aufweisen, daß Feinstaub über die Bypass-Leitung 8 aus dem Domraum 6 ausgeschleust werden kann, hier erfolgt, wie beim Ausführungsbeispiel der Fig. 1, eine Abscheidung über einen außen liegenden Feinstaubfilter 9.

Natürlich sind die beschriebenen Ausführungsbeispiele der Erfindung noch in vielfacher Hinsicht abzuändern, ohne den Grundgedanken zu verlassen, so kann beispielsweise eine Kombination der Gestaltung gemäß Fig. 1 und 2 vorgenommen werden u. dgl. mehr.

## Patentansprüche

1. Verfahren zur Feinstaubausschleusung aus einem Wirbelschichtreaktor zur Oxichlorierung von Ethylen,
**dadurch gekennzeichnet,**
**daß** der Feinstaub innerhalb des Reaktors über Filterkerzen, insbesondere Sintermetallfilterkerzen, ausgeschleust und
das Reaktionsgasgemisch zur Quenche aus dem Reaktordom geleitet wird, wobei ein Teilstrom als Bypass-Strom mit einem vorbestimmten Feinstaubanteil unterhalb einer vorbestimmten Korngröße neben dem Hauptstrom aus dem Reaktor ausgeschleust wird.

2. Verfahren nach Anspruch 1,
**dadurch gekennzeichnet,**
**daß** der Hauptstrom und der Bypass-Strom aus getrennten Domräumen des Reaktors ausgeschleust werden.

3. Verfahren nach Anspruch 1 oder 2,
**dadurch gekennzeichnet,**
**daß** der Bypass-Strom nach Analyse einer Katalysatorprobe und/oder Änderung der Wärmeübertragung und/oder Verschlechterung des Fluidisierungsverhaltens zu- bzw. abgeschaltet wird.

4. Wirbelschichtreaktor zur Oxichlorierung von Ethylen unter Einsatz eines dem Abrieb unterworfenen Katalysatorgranulates,
**dadurch gekennzeichnet,**
**daß** im Dom (6) des Reaktors (1) wenigstens eine Grundplatte (4) mit Sintermetallfilterkerzen (5) vorgesehen ist, wobei ggf. die Filterkerzen in den oberen Bereich der Wirbelschicht (2) eintauchen, und daß der Domraum (6) oberhalb der die Filterkerzen (5) auf seiner Unterseite tragenden Platte in wenigstens zwei Kammern (6,6a) mit je einem Ausgang (11) für einen Hauptstrom zur Quenche und einem Bypass-Strom (8) geteilt ist.

5. Wirbelschichtreaktor nach Anspruch 4,
**dadurch gekennzeichnet,**
**daß** die dem Bypass (8) zugeordneten Filterelemente (5a) eine gegenüber den Feinstaub-Filterkerzen (5) abweichende Porengröße aufweisen zum gezielten Durchlaß von Feinstaubanteilen.

6. Wirbelschichtreaktor nach Anspruch 4 oder 5,
**dadurch gekennzeichnet,**
**daß** das Verhältnis an Feinstaub durchlassenden Filterelementen (5a) zu den Feinstaub rückhaltenden Filterkerzen (5) im Bereich von 1:9 liegt.

7. Wirbelschichtreaktor nach Anspruch 4 oder einem der folgenden Ansprüche,
**dadurch gekennzeichnet,**
**daß** die Grundplatte (4) mit einer Abreinigungseinrichtung mittels Druckgaspulsen versehen ist.

## Claims

1. Process for removing fine dust from a fluidised bed reactor for the oxychlorination of ethylene,
**characterised in that** the fine dust inside the reactor is removed via filter candles, more particularly sintered metal filter candles, and the reaction gas mix is passed out of the reactor dome for quenching, with a component stream in the form of a bypass stream with a predetermined proportion of fine dust below a predetermined particle size being removed from the reactor along with the principal stream.

2. Process according to claim 1, **characterised in that** the principal stream and the bypass stream are removed from separate dome areas of the reactor.

3. Process according to claim 1 or 2, **characterised in that** the bypass stream is connected and disconnected following analysis of a catalyst sample and/or a modification of the heat transfer and/or a deterioration in the fluidising behaviour.

4. Fluidised bed reactor for the oxychlorination of ethylene using a granular catalyst that is subject to abrasion,
**characterised in that** at least one bottom plate (4) with sintered metal filter candles (5) is provided in the dome (6) of the reactor (1), and if appropriate the filter candles are immersed in the upper region of the fluidised bed (2), and that above the plate supporting the filter candles (5) the dome area (6) is divided on its underside into at least two. compartments (6, 6a) each with an exit (11) for a principal stream for quenching and with a bypass stream (8).

5. Fluidised bed reactor according to claim 4,
**characterised in that** the filter elements (5a) operatively associated with the bypass (8) present a deviating pore size in relation to the fine dust filter candles (5), so as to systematically allow through fine dust components.

6. Fluidised bed reactor according to claim 4 or 5,
**characterised in that** the ratio of filter elements (5a) that let through fine dust to the filter candles (5) that hold back fine dust is within the range 1 : 9.

7. Fluidised bed reactor according to claim 4 or any of the subsequent claims,
**characterised in that** the bottom plate (5) is provided with a dedusting device that utilises pulses of pressurised gas.

## Revendications

1. Procédé pour évacuer des poussières fines d'un réacteur à lit fluidisé pour l'oxychloration de l'éthylène,
**caractérisé en ce que**
les poussières fines sont évacuées par l'intermédiaire de bougies filtrantes, en particulier de filtres en métal fritté en forme de bougie, à l'intérieur du réacteur et **en ce que** le mélange réactionnel gazeux est conduit au refroidissement en dehors du réacteur, un flux partiel étant évacué du réacteur, indépendamment du flux principal, en tant que flux de dérivation, contenant une fraction de poussières fines prédéterminée en dessous d'une granulométrie prédéterminée.

2. Procédé selon la revendication 1,
**caractérisé en ce que**
le flux principal et le flux de dérivation sont évacués hors de zones distinctes du dôme du réacteur,

3. Procédé selon la revendication 1 ou 2
**caractérisé en ce que**
le flux de dérivation est activé ou désactivé après analyse d'un échantillon du catalyseur et/ou modification du transfert thermique et/ou altération du comportement de fluidification.

4. Réacteur à lit fluidisé pour l'oxychloration de l'éthylène avec mise en oeuvre d'un catalyseur en granulés soumis à une abrasion,
**caractérisé en ce que**
l'on prévoit dans le dôme (6) du réacteur (1) au moins un plateau (4) avec des filtres en métal fritté en forme de bougie (5), les bougies filtrantes plongeant éventuellement dans le lit fluidisé (2) et **en ce que** la zone du dôme (6), au-dessus de la plaque supportant, sur son côté envers, les bougies filtrantes (5), est divisée en au moins deux chambres (6, 6a) comportant chaque fois une sortie (11) pour un flux principal vers le refroidissement et un flux de dérivation (8).

5. Réacteur à lit fluidisé selon la revendication 4
**caractérisé en ce que**
les éléments filtrants (5a) associés à la dérivation (8) présentent une grosseur de pore différant par rapport aux filtres à poussières fines en forme de bougie (5) afin d'obtenir un passage ciblé des fractions de poussières fines.

6. Réacteur à lit fluidisé selon la revendication 4 ou 5,
**caractérisé en ce que**
le rapport entre les éléments filtrants laissant passer les poussières fines (5a) et les éléments filtrants retenant les poussières fines (5) est de l'ordre de 1:9

7. Réacteur à lit fluidisé selon la revendication 4 ou l'une quelconque des revendications suivantes,
**caractérisé en ce que**
les plateaux (4) sont équipés d'un dispositif de nettoyage au moyen d'impulsions de gaz comprimé.
